# EUROPEAN PATENT APPLICATION

(11) **EP 3 763 219 A1**
(43) Date of publication of application: **13.01.2021**
(21) Application number: 20174767.2
(22) Date of filing: 20.10.2008
(51) Int. Cl.: A23C 9/14, A23C 9/20, C11B 1/00, A23C 7/04, C11B 7/00, A23D 9/007, A23D 9/013, A23L 33/115, A61P 25/00, A61P 25/28

(54) **METHODS OF MAINTAINING OR INCREASING GROWTH OR COGNITIVE DEVELOPMENT**

(30) Priority: 19.10.2007 NZ 56270607
(62) Divisional of application: 08839768.2
(71) Applicant: Fonterra Co-Operative Group Limited, Auckland 1010 (NZ)
(72) Inventor: Hodgkinson, Steven Charles, Palmerston North (NZ); McJarrow, Christopher Paul, Palmerston North (NZ); Mitchell, Murray D., Palmerston North (NZ); Rowan, Angela Marie, Palmerston North (NZ); Todd, Joanne Margaret, Palmerston North (NZ); Vickers, Mark Hedley, Palmerston North (NZ)
(74) Representative: Forresters IP LLP

(57) **Abstract**

One or more complex lipids including gangliosides to achieve particular health benefits including maintaining or increasing cognitive development or maintaining or increasing growth in a foetal, infant or child subject.

## Description

### FIELD OF THE INVENTION

The present invention relates to using one or more complex lipids including gangliosides to achieve particular health benefits including maintaining or increasing cognitive development or maintaining or increasing growth in a foetal, infant or child subject.

This application is based on New Zealand provisional specifications NZ 562706 and NZ 562708, both incorporated herein by reference in their entirety.

### BACKGROUND

The composition of mammalian milk is specifically targeted to support normal growth and development of the infant or child (International Code of Marketing Breastmilk Substitutes, World Health Organisation, Geneva, 1981).

Maternal formulas, infant formulas, follow-on formulas, growing-up formulas, dietetic products and other dairy containing compositions are typically produced using non-human milk. However, the nutritional composition of human milk differs in some respects to that of non-human milk. Non-human whole milk such as cow, goat or sheep milk, contains a higher proportion of saturated fatty acids than human milk and has lower levels of linoleic acid and alpha-linolenic acid, and polyunsaturated fatty acids that are essential for normal growth and development. (Fox & McSweeney, 2006)

Standard maternal formulas, infant formulas, follow-on formulas and growing-up formulas among other products are typically produced using low-fat dairy products such as skim milk. Using a reduced-fat dairy product means allegedly undesirable components in milk fat are not included in the final product, but it also means that complex lipids such as phospholipid and (glyco)sphingolipid levels are significantly lower than those in human milk. (Sanchez-Diaz et al 1997; Pan and Imuzi 2000)

Optimal cognitive development and growth is a key part of infant and child development. Clearly, impaired cognitive development will have significant effects on quality of life. Additionally, restricted growth has been shown to have detrimental effects on long-term health. Therefore, any agent shown to increase cognitive development or maintain healthy growth will have wide benefits for infants and children. (Bryan et al 2004)

Complex lipids such as gangliosides are reported to have a range of potential functions because ganglioside profiles vary from one tissue to another (Rueda et al., 1998). The profile of individual ganglioside species is reported to change profoundly during development (Rosner, 2003) and gangliosides are reported to have beneficial effects on neural development (Rahmann, 1995) and are reported to be essential synaptic components and elicitors of neuronal migration and neurite outgrowth (Mendez-Otero & Santiago, 2003).

Ganglioside GMla is reported to cross the placenta in rats (Hungund et al, 1993) but definitive evidence of ganglioside transfer across the human placenta is lacking. Variation of gangliosides in human and bovine milk, and infant formulas is reported to potentially have some biological significance for neonatal brain development, allergies and infant growth (Pan et al, 2000; Rueda, 2007; Tram et al 1997).

Accordingly, it is an object of the present invention to provide means for maintaining or . increasing the cognitive development or growth of a subject, or to at least provide the public with a useful choice.

### SUMMARY OF THE INVENTION

In a first aspect the invention relates to one or more complex lipids for maintaining or increasing growth or maintaining or increasing cognitive development of a foetal, infant, or child subject.

In another aspect the invention relates to use of one or more complex lipids, such as
(i) one or more pure gangliosides, or
(ii) a composition comprising one or more gangliosides (such as a milk fat extract)
in the manufacture of a formulation for maintaining or increasing growth or maintaining or increasing cognitive development of a foetal, infant, or child subject wherein the formulation is orally administered to a mother during gestation.

In another aspect the invention relates to the use of one or more complex lipids in the manufacture of a formulation for maintaining or increasing growth or maintaining or increasing cognitive development of a foetal, infant, or child subject.

In another aspect the invention relates to a method for maintaining or increasing growth or maintaining or increasing cognitive development of a foetal, infant, or child subject by administering a composition comprising one or more complex lipids to a foetal, infant, or child subject in need thereof.

In another aspect the invention relates to a method of using one or more complex lipids such as one or more gangliosides for maintaining or increasing growth or maintaining or increasing cognitive development of a foetal subject, the method comprising providing a pregnant mother with a composition comprising one or more complex lipids such as one or more gangliosides and informing the mother that the composition will maintain or increase growth or maintain or increase cognitive development of the foetal subject.

In another aspect the invention relates to a method of using one or more complex lipids such as one or more gangliosides for maintaining or increasing growth or maintaining or increasing cognitive development in a subject, the method comprising providing a subject with a composition comprising one or more complex lipids such as one or more gangliosides and informing the subject that the composition will maintain or increase growth or maintain or increase cognitive development of the subject.

The following embodiments may relate to any of the above aspects.

In one embodiment, the one or more complex lipids are administered to a mother during gestation and the growth is brain weight of a foetal subject or brain ganglioside content of a foetal subject.

In one embodiment, the one or more complex lipids are administered to an infant or child subject and the growth is one or more of body weight, body length, and bone mineral density.

Preferably the ganglioside is GM3. Alternatively the ganglioside is GD3. More preferably the ganglioside comprises GM3 and GD3. In other embodiments, the composition comprising one or more gangliosides comprises one or more gangliosides selected from GM1, GM2, GM3, GM4, GD1, GD2, GD3, GT1, GT2, GT3, GQ1, and GP1, and any one or more of the "a", "b", or "c" derivatives where they exist, and any combination of any two or more thereof.

Preferably the composition comprising one or more complex lipids comprises at least about 0.1% gangliosides w/w on a dry basis. More preferably the composition comprising one or more complex lipids comprises at least 0.2% gangliosides w/w on a dry basis.

Alternatively, a formulation useful herein comprises at least about 1, 2, 3, 4, 5, 6, 7, 8, 9, 10, 11, 12, 13, 14, 15, 16, 18, 19, 19 or 20 mg, preferably at least about 5 mg or at least about 10 mg gangliosides /100g formulation, and useful ranges may be chosen between any of these values (for example, about 1 to about 20, about 2 to about 20, about 3 to about 20, about 1 to about 10, about 2 to about 10, or about 3 to about 10 mg). Preferably the formulation comprises about 5 mg to about 20 mg gangliosides / 100g formulation.

Alternatively the formulation is formulated to provide at least about 1, 2, 3, 4, 5, 6, 7, 8, 9, 10, 11, 12, 13, 14, 15, 16, 18, 19, 19 or 20 mg, preferably at least about 7.5 mg gangliosides per day to the mother, and useful ranges may be chosen between any of these values (for example, about 1 to about 20, about 2 to about 20, about 3 to about 20, about 1 to about 10, about 2 to about 10, or about 3 to about 10 mg). Preferably the formulation is formulated to provide about 7.5 mg to about 10 mg gangliosides per day to the mother.

In one embodiment, a formulation useful herein may comprise:
(a) 80-99.9% of a milk powder selected from whole milk powder, skim milk powder, milk protein concentrate (MPC), milk protein isolate (MPI), and whey protein such as a WPC or WPI
(b) 0-20% lipid such as milk fat or one or more vegetable oil
(c) 0-25% sugars or carbohydrate ingredient
(d) 0.1-0.5% vitamin and mineral mix
(e) 0-5% flavour ingredients, and
(f) 0-5% ganglioside ingredient.

Preferably the complex lipid comprises one or more phospholipids, or one or more sphingolipids, or one or more sphingomyelins or derivatives thereof, or .one or more ceramides, or one or more gangliosides, or a combination of any two or more thereof. Preferably the ganglioside is GM3. Alternatively the ganglioside is GD3. Alternatively the ganglioside comprises a mixture of at least GM3 and GD3.

In preferred embodiments, the formulation is a liquid (concentrate or ready-to-drink) or powdered maternal formula, infant formula, follow-on formula, growing-up formula or dietetic product.

In some embodiments the composition comprising one or more gangliosides (such as a milk fat extract) comprises at least about 0.1, 0.2, 0.3, 0.4, 0.5, 0.6, 0.7, 0.8, 0.9, 1.0, 1 5, 2, 2.5, 3, 3.5, 4, 4.5, 5, 5.5, 6, 6.5, 7, 7.5 8, 8.5, 9, 9.5, 10, 15, 20, 25, 30, 35, 40, 45, 50, 55, 60, 65, 70, 75, 80, 85, 90, 95, 99 or 99.5% by weight total lipid, and useful ranges may be selected between any of these values (for example, about 5 to about 95%, about 10 to about 95%, about 15 to about 95%, about 20 to about 95%, about 25 to about 95%, about 30 to about 95%, about 35 to about 95%, about 40 to about 95%, about 45 to about 95%, about 50 to about 95%, about 5 to about 99%, about 10 to about 99%, about 15 to about 99%, about 20 to about 99%, about 25 to about 99%, about 30 to about 99%, about 35 to about 99%, about 40 to about 99%, about 45 to about 99%, about 50 to about 99%, about 5 to about 70%, about 10 to about 70%, about 15 to about 70%, about 20 to about 70%, about 25 to about 70%, about 30 to about 70%, about 35 to about 70%, about 40 to about 70%, about 45 to about 70%, and about 50 to about 70% by weight total lipid).

In some embodiments the composition comprising one or more gangliosides (such as a milk fat extract) comprises at least about 0.1, 0.2, 0.3, 0.4, 0.5, 0.6, 0.7, 0.8, 0.9, 1.0, 1 5, 2, 2.5, 3, 3.5, 4, 4.5, 5, 5.5, 6, 6.5, 7, 7.5 8, 8.5, 9, 9.5, 10, 15, 20, 25, 30, 35, 40, 45, 50, 55, 60, 65, 70, 75, 80, 85, 90, 95, 99 or 99.5% by weight phospholipid, and useful ranges may be selected between any of these values (for example, about 5 to about 95%, about 10 to about 95%, about 15 to about 95%, about 20 to about 95%, about 25 to about 95%, about 30 to about 95%, about 35 to about 95%, about 40 to about 95%, about 45 to about 95%, about 50 to about 95%, about 5 to about 99%, about 10 to about 99%, about 15 to about 99%, about 20 to about 99%, about 25 to about 99%, about 30 to about 99%, about 35 to about 99%, about 40 to about 99%, about 45 to about 99%, about 50 to about 99%, about 5 to about 70%, about 10 to about 70%, about 15 to about 70%, about 20 to about 70%, about 25 to about 70%, about 30 to about 70%, about 35 to about 70%, about 40 to about 70%, about 45 to about 70%, and about 50 to about 70% by weight phospholipid).

In some embodiments the composition comprising one or more gangliosides (such as a milk fat extract) comprises at least about 0.1, 0.2, 0.3, 0.4, 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30% by weight of one or more phospholipids selected independently from phosphatidylcholine, phosphatidylethanolamine, sphingomyelin, phosphatidylserine, and phosphatidylinositol, and useful ranges may be selected between any of these values (for example, about 0.1 to about 30%, about 0.5 to about 30%, about 1 to about 30%, about 2 to about 30%, about 3 to about 30%, about 4 to about 30%, about 5 to about 30%, about 10 to about 30%, about 15 to about 30%, about 20 to about 30%, about 0.1 to about 5%, about 0.5 to about 5%, about 1 to about 5%, about 2 to about 5%, about 3 to about 5%, about 0.1 to about 10%, about 0.5 to about 10%, about 1 to about 10%, about 2 to about 10%, about 3 to about 10%, about 4 to about 10%, about 5 to about 10%, about 6 to about 10%, about 0.1 to about 20%, about 0.5 to about 20%, about 1 to about 20%, about 2 to about 20%, about 3 to about 20%, about 4 to about 20%, about 5 to about 20%, about 10 to about 20%, about 15 to about 20% by weight of one or more phospholipids selected independently from phosphatidylcholine, phosphatidylethanolamine, sphingomyelin, phosphatidylserine, and phosphatidylinositol).

In some embodiments the composition comprising one or more gangliosides (such as a milk fat extract) comprises at least about 0.1, 0.2, 0.3, 0.4, 0.5, 0.6, 0.7, 0.8, 0.9, 1.0, 1 5, 2, 2.5, 3, 3.5, 4, 4.5, 5, 5.5, 6, 6.5, 7, 7.5 8, 8.5, 9, 9.5, 10, 15, 20, 25, 30, 35, 40, 45, 50, 55, 60, 65, 70, 75, 80, 85, 90, 95 or 99.5% by weight ganglioside, and useful ranges may be selected between any of these values (for example, about 5 to about 95%, about 10 to about 95%, about 15 to about 95%, about 20 to about 95%, about 25 to about 95%, about 30 to about 95%, about 35 to about 95%, about 40 to about 95%, about 45 to about 95%, about 50 to about 95%, about 10 to about 70%, about 15 to about 70%, about 20 to about 70%, about 25 to about 70%, about 30 to about 70%, about 35 to about 70%, about 40 to about 70%, about 45 to about 70%, and about 50 to about 70% by weight phospholipid). In one embodiment the composition comprising one or more gangliosides comprises GD3 or GM3 or a combination thereof. In one embodiment, the composition comprising one or more gangliosides comprises one or more gangliosides selected from GM1, GM2, GM3, GM4, GD1, GD2, GD3, GT1, GT2, GT3, GQ1, and GP1, and any one or more of the "a", "b", or "c" derivatives where they exist, and any combination of any two or more thereof.

In some embodiments the composition comprising one or more gangliosides (such as a milk fat extract) comprises at least about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 3, 4, 5, 6, 7, 8, 9, 10, 11, 12, 13, 14, 15, 16, 17, 18, 19, 20, 21, 22, 23, 24, 25, 26, 27, 28, 29 or 30% by weight of one or more gangliosides selected independently from GD3 and GM3, and useful ranges may be selected between any of these values (for example, about 0.1 to about 30%, about 0.5 to about 30%, about 1 to about 30%, about 2 to about 30%, about 3 to about 30%, about 4 to about 30%, about 5 to about 30%, about 10 to about 30%, about 15 to about 30%, about 20 to about 30%, about 0.1 to about 5%, about 0.5 to about 5%, about 1 to about 5%, about 2 to about 5%, about 3 to about 5%, about 0.1 to about 10%, about 0.5 to about 10%, about 1 to about 10%, about 2 to about 10%, about 3 to about 10%, about 4 to about 10%, about 5 to about 10%, about 6 to about 10%, about 0.1 to about 20%, about 0.5 to about 20%, about 1 to about 20%, about 2 to about 20%, about 3 to about 20%, about 4 to about 20%, about 5 to about 20%, about 10 to about 20%, about 15 to about 20% by weight of one or more gangliosides selected independently from GD3 and GM3.

In one embodiment, the composition comprising one or more gangliosides comprises about 15% to about 99% by weight total lipid, about 1% to about 80% by weight phospholipid, about 1% to about 25% by weight phosphatidylcholine, about 0.1% to about 15% by weight phosphatidylinositol, about 0.1% to about 15% by weight phosphatidylserine, about 1% to about 30% by weight phosphatidylethanolamine, about 0.5% to about 25% by weight sphingomyelin, and about 0.1 to about 10% by weight ganglioside. In some embodiments the composition comprising one or more gangliosides comprises about 1% to about 60% by weight lactose, about 1% to about 15 % by weight lactose or about 50% to about 65% by weight lactose.

In alternative embodiments, the composition comprising one or more gangliosides comprises about 20% to about 40 % by weight total lipid, about 5% to about 25% by weight phospholipid, and amounts of one or more phospholipids as described above. In other alternative embodiments, the composition comprising one or more gangliosides comprises about 70% to about 99 % by weight total lipid, about 25% to about 80% by weight phospholipid, and amounts of one or more phospholipids as described above.

In still further alternative embodiments, the composition comprising one or more gangliosides comprises about 0.1% to about 10%, about 0.1% to about 2.5%, or about 3% to about 10% by weight of one or more gangliosides, preferably independently selected from GD3 and GM3.

In another embodiment, the composition comprising one or more gangliosides comprises about 15 to 40% total lipid, about 10 to 25% phospholipid, about 1% to about 6% phosphatidylcholine, about 1% to about 6% phosphatidylinositol, about 1% to about 6% phosphatidylserine, about 1% to about 6% phosphatidylethanolamine, and about 1% to about 3% sphingomyelin. In a preferred embodiment, the composition comprising one or more gangliosides comprises at least about 3% to about 6% myristic acid (14:0), at least about 12% to about 20% palmitic acid (16:0), at least about 0.5% to about 3% palmitoleic acid (16:1), at least about 0.1% to about 1.5% margaric acid (17:0), at least about 13% to about 20% stearic acid (18:0), at least about 28% to about 35% oleic acid (18:1), at least about 3% to about 5% linoleic acid (18:2) and at least about 0.5% to about 2.5% linolenic (18:3). In some embodiments, the composition comprising one or more gangliosides comprises about 0.1% to about 2.5% ganglioside GD3, about 0.1% to about 1% ganglioside GM3, or both.

In one embodiment the composition comprising one or more gangliosides comprises one or more phosphatidylethanolamines, one or more phosphatidylinositols, one or more phosphatidylserines, one or more phosphatidylcholines, one or more sphingolipids (including one or more sphingomyelins, one or more dihydrosphingomyelins, one or more ceramides, one or more cerebrosides, or one or more gangliosides, or any combination of any two or more thereof), one or more lysophospholipids (phospholipids with one fatty acid lost), or any combination of any two or more thereof.

In some embodiments the milk fat extract comprises
(a) about 15 to about 25% w/w lipid, about 5 to about 15% w/w phospholipid, and about 0.1 to about 1% w/w ganglioside, or
(b) about 15 to about 25% w/w lipid, about 5 to about 15% w/w phospholipid, about 1 to about 5% w/w phosphatidylcholine, about 0.1 to 2% w/w phosphatidylinositol, about 0.5 to about 2% w/w phosphatidylserine, about 1.5 to about 6% w/w phosphatidylethanolamine, about 1 to about 5% w/w sphingomyelin, and about 0.1 to about 1% w/w ganglioside, or
(c) about 25 to about 45% w/w lipid, about 10 to about 25% w/w phospholipid, and about 0.1 to about 2.0% w/w ganglioside, or
(d) about 25 to about 45% w/w lipid, about 10 to about 25% w/w phospholipid, about 1 to about 5% w/w phosphatidylcholine, about 0.1 to 2% w/w phosphatidylinositol, about 0.5 to about 2% w/w phosphatidylserine, about 1.5 to about 6% w/w phosphatidylethanolamine, about 1 to about 5% w/w sphingomyelin, and about 0.1 to about 2.0% w/w ganglioside, or
(e) about 12 to about 32% w/w lipid, about 5 to about 25% w/w phospholipid, and about 0.1 to about 2.0% w/w ganglioside, or
(f) about 12 to about 32% w/w lipid, about 5 to about 25% w/w phospholipid, about 2 to about 8 % w/w phosphatidylcholine, about 0.5 to 3% w/w phosphatidylinositol, about 1 to about 3.5% w/w phosphatidylserine, about 1 to about 10% w/w phosphatidylethanolamine, about 1 to about 8% w/w sphingomyelin, and about 0.5 to about 2.5% w/w ganglioside, or
(g) about 80 to about 99% w/w lipid, about 20 to about 75% w/w phospholipid, and about 0.5 to about 5% w/w ganglioside, or
(h) about 80 to about 99% w/w lipid, about 20 to about 75% w/w phospholipid, about 2 to about 22 % w/w phosphatidylcholine, about 1 to about 10% w/w phosphatidylinositol, about 1 to about 10% w/w phosphatidylserine, about 5 to about 30% w/w phosphatidylethanolamine, about 1 to about 20% w/w sphingomyelin, and about 0.5 to about 5% w/w ganglioside, or
(i) about 90 to about 99% w/w lipid, about 20 to about 40% w/w phospholipid, and about 0.5 to about 5% w/w ganglioside, or
(j) about 80 to about 99% w/w lipid, about 60 to about 80% w/w phospholipid, and about 0.5 to about 5% w/w ganglioside, or
(k) about 15 to about 45% w/w lipid, about 8 to about 25% w/w phospholipid, and about 0.1 to about 5 % w/w ganglioside, or
(l) about 15 to about 45% w/w lipid, about 8 to about 25% w/w phospholipid, about 1 to about 5% w/w phosphatidylcholine, about 1 to about 5% w/w phosphatidylinositol, about 2 to about 8% w/w phosphatidylserine, about 2 to about 8% w/w phosphatidylethanolamine, about 0.5 to about 5% w/w sphingomyelin, and about 0.1 to about 5% w/w ganglioside, or
(m) about 50 to about 99% w/w lipid, about 15 to about 60% w/w phospholipid, and about 1 to about 10% w/w ganglioside, or
(n) about 50 to about 99% w/w lipid, about 15 to about 60% w/w phospholipid, about 1 to about 10% w/w phosphatidylcholine, about 1 to about 15% w/w phosphatidylinositol, about 1 to about 20% w/w phosphatidylserine, about 1 to about 20% w/w phosphatidylethanolamine, about 1 to about 10% w/w sphingomyelin, and about 0.1 to about 10% w/w ganglioside.

In one embodiment, a formulation useful herein comprises at least about 0.1, 0.2, 0.5, 1, 5, 10, 15, 20, 25, 30, 35, 40, 45, 50, 55, 60, 65, 70, 75, 80, 85, 90, 95, 99, 99.5, 99.8 or 99.9% by weight of the composition comprising one or more gangliosides and useful ranges may be selected between any of these foregoing values (for example, from about 0.1 to about 50%, from about 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%, from about 40 to about 50%, from about 45 to about 50%, from about 0.1 to about 60%, from about 0.2 to about 60%, from about 0.5 to about 60%, from about 1 to about 60%, from about 5 to about 60%, from about 10 to about 60%, from about 15 to about 60%, from about 20 to about 60%, from about 25 to about 60%, from about. 30 to about 60%, from about 35 to about 60%, from about 40 to about 60%, from about 45 to about 60%, from about 51 to about 60%, from about 51 to about 60%, from about 0.1 to about 70%, from about 0.2 to about 70%, from about 0.5 to about 70%, from about 1 to about 70%, from about 5 to about 70%, from about 10 to about 70%, from about 15 to about 70%, from about 20 to about 70%, from about 25 to about 70%, from about 30 to about 70%, from about 35 to about 70%, from about 40 to about 70%, from about 45 to about 70%, from about 51 to about 70%, from about 0.1 to about 80%, from about 0.2 to about 80%, from about 0.5 to about 80%, from about 1 to about 80%, from about 5 to about 80%, from about 10 to about 80%, from about 15 to about 80%, from about 20 to about 80%, from about 25 to about 80%, from about 30 to about 80%, from about 35 to about 80%, from about 40 to about 80%, from about 45 to about 80%, from about 51 to about 80%, from about 0.1 to about 90%, from about 0.2 to about 90%, from about 0.5 to about 90%, from about 1 to about 90%, from about 5 to about 90%, from about 10 to about 90%, from about 15 to about 90%, from about 20 to about 90%, from about 25 to about 90%, from about 30 to about 90%, from about 35 to about 90%, from about 40 to about 90%, from about 45 to about 90%, from about 51 to about 90%, from about 0.1 to about 99%, from about 0.2 to about 99%, from about 0.5 to about 99%, from about 1 to about 99%, from about 5 to about 99%, from about 10 to about 99%, from about 15 to about 99%, from about 20 to about 99%, from about 25 to about 99%, from about 30 to about 99%, from about 35 to about 99%, from about 40 to about 99%, from about 45 to about 99%, and from about 51 to about 99%). Hydrolysed forms of the composition comprising one or more gangliosides may be used, where hydrolysis is performed using known methods to a desired degree of hydrolysis.

In one embodiment a formulation useful herein comprises at least about 0.001, 0.01, 0.05, 0.1, 0.15, 0.2, 0.3, 0.4, 0.5,1, 2, 3, 4, 5, 6, 7, 8, 9, 10,11, 12, 13, 14, 15, 16, 17, 18 or 19 grams of the composition comprising one or more gangliosides as described above and useful ranges may be selected between any of these foregoing values (for example, from about 0.01 to about 1 grams, about 0.01 to about 10 grams, about 0.01 to about 19 grams, from about 0.1 to about 1 grams, about 0.1 to about 10 grams, about 0.1 to about 19 grams, from about 1 to about 5 grams, about 1 to about 10 grams, about 1 to about 19 grams, about 5 to about 10 grams, and about 5 to about 19 grams).

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

Figures 1 to 3 show results of various testing parameters from the Morris Water Maze Task of Example 1 of Example 1. Data are mean ± SEM, n=16 per control (Blank gel) and Low dose gel groups, n=15 in the High dose gel treated group.
Figure 4 shows the results from the Novel Object Recognition Test of Example 1.
Figure 5 is a graph showing the postnatal growth of the rats in Example 2. N=16 per group, data are mean ± SEM.
Figure 6 is a graph showing the changes in ganglioside composition in rat pups in Example 4. Gangliosides GM1a, GD1a, GDlb and GT1b were significantly increased (p<0.05).

### DETAILED DESCRIPTION OF THE INVENTION

### 1. Definitions

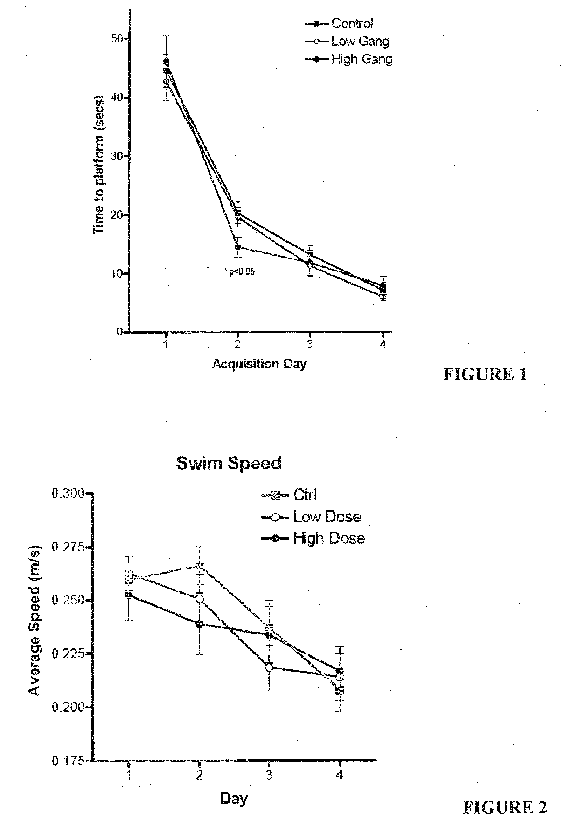

The term "beta-serum" means an aqueous dairy ingredient separated from dairy streams containing greater than 60% fat that have been through phase inversion from an oil-in-water to a water-in-oil emulsion, as described below. Cream is the preferred starting material for the production of beta-serum. For example, beta-serum is produced during the production of butter-oil (also known as anhydrous milk fat or AMF) from cream as shown in Figure 2 of WO 2006/041316, incorporated herein by reference. Preferably the beta serum is dried; preferably dried beta-serum is a powder.

The term "comprising" as used in this specification and claims means "consisting at least in part of". When interpreting statements in this specification and claims which include that term, the features, prefaced by that term in each statement, all need to be present but other features can also be present. Related terms such as "comprise" and "comprised" are to be interpreted in the same manner.

The term "levels in foetal circulation" as used herein means foetal blood levels and/or foetal lymph levels and/or foetal tissue levels.

The term "complex lipid" as used in this specification means a lipid selected from the group consisting of phospholipids and sphingolipids including glycosphingolipids (both cerebrosides and gangliosides), ceramides and sphingomyelins. Different types of complex lipids are discussed in more detail below. Complex lipids may be found in milk and other dairy sources. Other sources of some complex lipids include any animal tissue but especially brain and nervous tissue, eggs, fish, deer velvet and plant lipids. Preferably the complex lipids used in the present invention are derived from a dairy ingredient. Suitable dairy ingredients include colostrum, milk, fractions of colostrum or fractions of milk. Preferably the dairy ingredient is derived from cows, buffalos, goats, sheep or human. Most preferably the dairy ingredient is cow-derived. Preferably the complex lipid is in the form of a milk fat extract.

An "effective amount" is the amount required to confer therapeutic effect. The interrelationship of dosages for animals and humans (based on milligrams per meter squared of body surface) is described by Freireich, et al. (1966). Body surface area can be approximately determined from height and weight of the subject. See, e.g., Scientific Tables, Geigy Pharmaceuticals, Ardley, New York, 1970, 537. Effective doses also vary, as recognized by those skilled in the art, dependent on route of administration, carrier usage, and the like.

The terms "increasing cognitive development" or "to increase cognitive development" are used interchangeably herein and refer to increasing the rate, ability, interest, willingness, or openess to learn, remember or apply knowledge. In some embodiments, "cognitive development" refers to brain weight and brain ganglioside content.

The terms "increasing or maintaining growth" or "to increase or maintain growth" are used interchangeably herein and refer to increasing or maintaining healthy growth which may refer to increasing or maintaining the absolute growth or rate of growth with reference to weight, length, or height, while not increasing adiposity or decreasing bone density.

The term "maternal formula" as used in this specification means a composition for pregnant woman to take during pregnancy. The term "infant formula" as used in this specification means a composition for infants aged between 0 days and 6 months old. The term "follow-on formula" as used in this specification means a composition for infants aged 6 months to 1 year. The term "growing up formula" as used in this specification means a compositions directed to infants and children aged 1 year upwards. Growing-up formula includes growing-up milk powders or GUMPs.

It will be appreciated by those skilled in the art that the age ranges for the different compositions: "infant formula", "follow-on formula" and "growing-up formula" can vary from child to child depending on the individual's development. These products may be in liquid form as concentrates or ready-to-drink liquids or provided as powder concentrates.

The term "dietetic product" means a product specially processed or formulated to satisfy particular dietary requirements which exist because of a particular physical or physiological condition and/or specific diseases and disorders and which are presented as such.

The term "milk fat extract" means an isolated extract of non-human mammalian milk fat where the phospholipid and ganglioside concentration of the extract is higher than the phospholipid and ganglioside concentration of naturally occurring non-human mammalian milk fat. Preferably the concentration of at least one phospholipid and at least one ganglioside in an extract useful herein is at least about 0.5, 1, 5, 10, 15, 20, 25, 30, 35, 40, 45, 50, 55, 60, 65, 70, 75, 80, 85, 90, 95, or 100% higher than the concentration in naturally occurring non-human mammalian milk fat, and useful ranges may be selected between these values. In alternative embodiments the concentration in the extract is higher than the concentration in whole milk, or in whole colostrum, or in cream from milk, or in cream from colostrum, or in anhydrous milk fat (AMF) from milk, or AMF from colostrum.

In a formulation useful herein, the formulation may comprise, consist essentially of, or consist of about 0.1, 0.5, 1, 5, 10, 15, 20, 25, 30, 35, 40, 45, 50, 55, 60, 65, 70, 75, 80, 85, 90, 95, 99, or 100% by weight of fresh, recombined or powdered whole milk or a milk derivative and useful ranges may be selected between any of these foregoing values (for example, from about 0.1 to about 50%, from about 0.2 to about 50%, from about 0.5 to about 50%, from about 1 to about 50%, from about 5 to about 50%, from about 10 to about 50%, from about 15 to about 50%, from about 20 to about 50%, from about 25 to about 50%, from about 30 to about 50%, from about 35 to about 50%, from about 40 to about 50%, and from about 45 to about 50%). The milk derivative is preferably selected from recombined, powdered or fresh skim milk, reconstituted whole or skim milk powder, skim milk concentrate, skim milk retentate, concentrated milk, ultrafiltered milk retentate, milk protein concentrate (MPC), milk protein isolate (MPI), calcium depleted milk protein concentrate (MPC), low fat milk, low fat milk protein concentrate (MPC), casein, caseinate, milk fat, cream, butter, ghee, anhydrous milk fat (AMF), buttermilk, butter serum, hard milk fat fractions, soft milk fat fractions, sphingolipid fractions, milk fat globule membrane fractions, phospholipid fractions, complex lipid fractions, colostrum, a colostrum fraction, colostrum protein concentrate (CPC), colostrum whey, an immunoglobulin fraction from colostrum, whey, whey protein isolate (WPI), whey protein concentrate (WPC), sweet whey, lactic acid whey, mineral acid whey, reconstituted whey powder, a composition derived from any milk or colostrum processing stream, a composition derived from the retentate or permeate obtained by ultrafiltration or microfiltration of any milk or colostrum processing stream, a composition derived from the breakthrough or adsorbed fraction obtained by chromatographic (including but not limited to ion and gel permeation chromatography) separation of any milk or colostrum processing stream, extracts of any of these milk derivatives including extracts prepared by multistage fractionation, differential crystallisation, solvent fractionation, supercritical fractionation, near supercritical fractionation, distillation, centrifugal fractionation, or fractionation with a modifier (e.g. soaps or emulsifiers), hydrolysates of any of these derivatives, fractions of the hydrolysates, and combinations of these derivatives, including combinations of hydrolysed and/or non-hydrolysed fractions. It should be understood that the source of these derivatives may be milk or colostrum or a combination thereof. It should also be understood that the milk fat may be provided as fresh, recombined or powdered whole milk, one or more milk derivatives as described above, or combinations thereof.

In one embodiment a formulation useful herein further comprises a pharmaceutically acceptable carrier. In another embodiment the formulation is or is formulated as a food, drink, food additive, drink additive, dietary supplement, nutritional product, medical food, enteral or parenteral feeding product, meal replacement, nutraceutical, medicament or pharmaceutical. In one embodiment the formulation is in the form of a tablet, a caplet, a pill, a hard or soft capsule or a lozenge. In one embodiment the formulation is in the form of a cachet, a dispensable powder, granules, a suspension, an elixir, a liquid, or any other form that can be added to food or drink, including for example water, milk or fruit juice. In one embodiment the formulation further comprises one or more constituents (such as antioxidants) which prevent or reduce degradation of the formulation during storage or after administration. These formulations may include any edible consumer product which is able to carry lipid. Examples of suitable edible consumer products include aqueous products, baked goods, confectionary products including chocolate, gels, ice creams, reconstituted fruit products, snack bars, food bars, muesli bars, spreads, sauces, dips, dairy products including yoghurts and cheeses, drinks including dairy and non-dairy based drinks, milk, milk powders, sports supplements including dairy and non-dairy based sports supplements, fruit juice, food additives such as protein sprinkles and dietary supplement products including daily supplement tablets. Suitable nutraceutical compositions useful herein may be provided in similar forms.

The term "oral administration" includes oral, buccal, enteral and intra-gastric administration.

The term "pharmaceutically acceptable carrier" is intended to refer to a carrier including but not limited to an excipient, diluent, auxiliary or combination thereof that can be administered to a subject as a component of a composition of the invention that does not reduce the activity of the composition and is not toxic when administered in doses sufficient to deliver an effective amount of the active ingredient. The formulations can be administered orally or nasally.

A "subject" is an animal, preferably a mammal, more preferably a mammalian companion animal or human. Preferred companion animals include cats, dogs and horses.

### 2. Complex lipids

Phospholipids are a class of lipids and a major component of cell membranes. Sphingolipids are also a class of lipids the most structurally diverse class of membrane lipids. Sphingolipids are lipids mostly comprising the 18-carbon base sphingosine (some other base lengths are found), which has an acyl group attached by an amide linkage to form a ceramide (Newburg, 1996). There are three main types of sphingolipids:
(1) Glycosphingolipids (sugar-containing sphingolipids), which may be further subdivided into cerebrosides and gangliosides.
(2) Ceramides (consist simply of a fatty acid chain attached through an amide linkage to sphingosine).
(3) Sphingomyelins (phosphorylcholine or phosphoroethanolamine molecule esterified to the 1-hydroxy group of a ceramide). Sphingomyelins are also phospholipids.

Dairy-derived complex lipids are discussed comprehensively by Fox and McSweeney, incorporated herein by reference.

### 3. Ganglioside structure

What distinguishes the gangliosides from the other glycosphingolipids is that they contain the sugar sialic acid, which is negatively charged at physiological pH.

A wide variety of ganglioside glycans can be formed by the combination of glucose (Glc), galactose (Gal), N-acetyl galactosamine (GalNAc) and sialic acid together. As the glycan size increases, so does the hydrophilicity of the glycan portion. The ceramide portion is hydrophobic, making the whole molecule amphiphilic.

The nomenclature most commonly employed, because of its simplicity compared with the IUPAC nomenclature is that of Svennerholm (Svennerholm, 1963), which relates to the glycan portion of the ganglioside. All gangliosides start with G, and the next letter describes the number of sialic acid residues present in the molecule (M = mono, D = di, T = tri, Q = quad etc.). The next part of the nomenclature is a number, which describes the number of non-sialic-acid sugars in the molecule (1 indicates four sugars other than the sialic acid/s linked, Gal-GalNAc-Gal-Glc-ceramide; 2 indicates three sugars, GalNAc-Gal-Glc-ceramide; and 3 indicates two sugars, Gal-Glc-ceramide). Sometimes, there is a lower case letter attached at the end to designate where the sialic acids are attached, which is usually to galactose or another sialic acid. To add to the already complex glycan, further complexity is derived by the possible addition of fucose or modification of the sialic acid hydroxyl groups with additions of acetate (or other groups). Acetylation (4-, 7- or 9-O-acetylation) has major effects on bioactivity or recognition by other molecules such as proteins. Even further complexity is realised with the sialic acids because, although the most common sialic acids are N-acetyl neurammic acid (NANA) and N-glycolyl neuraminic acid (NGNA), more than 30 have been shown to exist in nature (Schauer, 2004).

Small structural changes in gangliosides can elucidate markedly different biological activities. An example of this is the binding of cholera toxin to gangliosides, the toxin is specific for one ganglioside, ganglioside GM1. The addition of a further NANA molecule (GD1a) or the loss of a galactose (GM2) leads to negligible binding. As mentioned above the O-acetylation effects the biological activity, for example the difference in effect between GD3 and 9-O-acetyl GD3 on neuronal out growth where the later promotes but the former does not. The former examples are changes in the glycan structure, recent work suggests that even changes in the fatty acid bound to the sphingosine influences activity. Thus one skilled in the art would not assume that biological activity attributed to one ganglioside would be attributed to another given the specificity of activity to specific ganglioside structures.

Gangliosides can be measured by a number of techniques. Ganglioside measurement may be done by measuring Lipid Bound Sialic Acid (LBSA) or individual species quantified by Thin Layer Chromatography (TLC), High Performance Liquid Chromatography with UV detection (HPLC-UV) or by liquid chromatography linked to mass spectrometry. A number of techniques can be employed to extract gangliosides from a variety of materials and these are well known to those skilled in the art.

Gangliosides useful herein include any one or more of GM1, GM2, GM3, GM4, GD1, GD2, GD3, GT1, GT2, GT3, GQ1, and GP1, and any of the "a", "b", or "c" derivatives where they exist, and any combination of any two or more thereof. Structure and synthesis of gangliosides is reviewed by Rosner, 2003, incorporated herein by reference.

### 4. Increasing cognitive development

Optimal cognitive development is a key part of infant and child development. Therefore any agent shown to increase cognitive development will have wide benefits for infants and children.

As outlined earlier, the terms "increasing cognitive development" or "to increase cognitive development" are used interchangeably herein and refer to increasing the rate, ability, interest, willingness, or openess to learn, remember or apply knowledge.

A wide variety of methods to assess cognitive development are well known to those skilled in the art. It will be apparent that particular methods may be preferred depending on the nature of the cognition to be assessed, the characteristics or identity (such as but not limited to the species, age, health or wellbeing) of the subject, or other factors as may be applicable. For example, methodology useful for the assessment of cognitive development in non-human subjects includes the Morris Water Maze Test and the Novel Object Recognition Task Test, as described in Example 1. Methodology useful for the assessment of cognitive development in human subjects includes the tools summarised in Table 1.

**Table 1 - Methods for assessment of human development**

| **Parameter** | **Tool used** | **Age group** | **Components of tool** | **Reference** |
|---|---|---|---|---|
| Cognitive and Motor Development. | Bayley Scales of Infant Development, Version 2, | 0-3 years | Global assessments of cognitive and motor development assesses the motor (fine and gross), language (receptive and expressive), and cognitive development | The Essentials of Bayley Scales of Infant Development II Assessment, Maureen M. Black, Kathleen Matula. New York: John Wily, 1999. ISBN: 978-0-471-32651-9 |
| Intelligence Quotient | Weschler Preschool & primary scale | 2.6-7.3 years | Verbal comprehension | WPPST (Wechsler Preschool and Primary Scale of Intelligence - Third Edition, 2002) published: Harcourt Assessment, David Wechsler |
| Memory | Children's Memory Scale | 5 - 8 years | 1. Attention and working memory | Children's Memory Scale (CMS) 1997, Morris Cohen |
| | | | 2. Verbal and Visual memory | |
| | | | 3. Short Delay and long delay | |
| | | | 4. Recall and recognition | |
| | | | 5. Learning Characteristics | |
| Development | Denver Developmen tal Materials | 0-6 years | General childhood development | www.denverii.com |
| Executive functioning | Wisconsin card sorting test | 5+ years | 1. Preservative thinking | Wisconsin Card Sorting Test: |
| | | | 2. Assess abstract reasoning | Computer Version 4 (WCST: CV4), Robert K. Heaton. |
| Academic Achievement | School Report Cards | 4-7 years | School Performance | Report Cards (Academic Performance in School Setting), Young Children Achievement |
| | | | | Test,Wayne P. Hresko |

### 5. Enhancing growth

Optimal growth is a key part of infant and child development. Restricted growth has been shown to have detrimental effects on long-term health and cognitive development. Therefore any agent shown to increase or maintain healthy growth will have wide benefits for infants and children.

As outlined earlier, the terms "increasing or maintaining growth" or "to increase or maintain growth" are used interchangeably herein and refer to increasing or maintaining healthy growth which may refer to increasing or maintaining the absolute growth or rate of growth with reference to weight, length, or height, while not increasing adiposity or decreasing bone density. These terms also refer to increasing bone mineral density and/or brain weight.

### 6. Isolation of complex lipids

Extracts or fractions containing higher levels of complex lipids than natural milk may be prepared in a number of ways. These include the extraction of milk or milk powder with chloroform/methanol mixtures (for an example see Martin et al., 2001) or Tetrahydrofuran (Neeser et al., 1991) or sub critical extraction with Dimethyl ether (WO 2006/041316A). Extraction of complex lipids from other tissues such as mammalian, marine and plant sources, including brain, neural tissue, liver, fish blood, egg, and plant materials has been achieved by a wide range of methodologies known to those skilled in the art (one such example is given in Svennerholm et al., 1994). Gangliosides may also be produced synthetically or semi-synthetically. Gangliosides useful herein include any one or more of GM1, GM2, GM3, GM4, GD1, GD2, GD3, GT1, GT2, GT3, GQ1, and GP1, and any of the "a", "b", or "c" derivatives where they exist, and any combination of any two or more thereof. Structure and synthesis of gangliosides is reviewed by Rosner, 2003, incorporated herein by reference.

Examples of extracts useful according to the invention include any "high fat" milk fraction for example: cream, butter, ghee, anhydrous milk fat (AMF), buttermilk, butter serum, beta serum, hard milk fat fractions, soft milk fat fractions, milk fat globule membrane fractions, and combinations thereof, and hydrolysates thereof.

Milk fat is discussed comprehensively by Fox and McSweeney (2006), hereby incorporated by reference. In addition to lipids, milk fat includes vitamins, sterols, and minor components. See Chapter 1, Composition and Structure of Bovine Milk Lipids, Fox and McSweeney, for a description of naturally occurring bovine milk fat. Fractionation of milk fat is discussed in by Bylund, 1995, Illingworth, 2002, and Rombaut et al, 2006(b), all hereby incorporated by reference. Seasonal variation of milk fat is discussed by Fox and McSweeney (2006).

Examples of sources of complex lipids useful herein include cream (typically about 20 to about 40% fat by weight, preferably about 40% fat by weight), butter, ghee, anhydrous milk fat (AMF) (typically produced by phase inversion of cream or dehydration of butter), buttermilk, butter serum, beta serum, hard milk fat extracts, soft milk fat extracts, sphingolipid extracts, milk fat globule membrane extracts, milk fat globule membrane lipid extracts, phospholipid extracts, and complex lipid (lipids that yield 3 or more types of hydrolysis product per molecule) extracts, and combinations thereof, and hydrolysates thereof.

Buttermilk, butter serum, and beta serum are discussed by Bylund, 1995, Rombaut et al, 2005, Rombaut et al, 2006(a), Rombaut et al, 2006(b), and published international application WO 2006/041316, for example, all incorporated herein by reference. Buttermilk is a term used to describe the aqueous liquid phase obtained from traditional butter production using a butter making process which may be a batch (churn) process or a continuous (Fritz) process. Buttermilk is also a term used to describe the aqueous by-product produced by the cream concentration step of the traditional method of producing AMF from cream. This traditional method involves concentration then phase inversion of cream to produce oil that is further concentrated and polished to produce AMF. Finally, buttermilk is also a term used to describe a combination of the secondary skim and beta serum by-products of a two-serum process for AMF production - see for example, Bylund (1995) and published international application WO 2006/041316 (see Figure 2) that describe this process in detail. In that two-serum process, the by-product from the cream concentration step is further separated to produce secondary skim and the by-product from the oil concentration step is further separated to produce beta-serum. In the first two instances, the buttermilk is produced before any phase inversion has occurred. In the third instance, the buttermilk is a combination of secondary skim produced before phase inversion and beta serum produced after phase inversion. Concentration and polishing in these processes is typically achieved by centrifugation. Phase inversion is typically achieved by homogenisation. It should be understood that the source of these dairy lipid extracts may be milk or colostrum or a combination thereof. Useful starting materials for fractionation include cream, AMF, butter milk, butter serum, or beta serum, from milk or colostrum or a combination thereof.

Multistage fractionation of milk fat may be carried out by differential crystallisation. Milk fat extracts are heated to a set temperature and the crystallised or solid ("stearin" - hard fraction) and liquid ("olein" - soft fraction) fractions are separated. Multi-step fractionation refers to re-fractionation in a subsequent step of a product of a previous fractionation step. Successive soft fractions may be produced by fractionating parent soft fractions into soft and hard sub-fractions.

Other fractionation methods include phase inversion, interesterification, glycerolysis, solvent fractionation (such as with ethanol, water, or acetone, used alone or sequentially), supercritical fractionation (see Astaire, et al, 2003, for example), near critical fractionation (see WO 2004/066744, for example), distillation, centrifugal fractionation, suspension crystallisation, dry crystallisation, fractionation with a modifier (e.g. soaps or emulsifiers), ultra-filtration, micro-filtration, and any process for fractionation of lipid known in the art, and combinations of these methods, all as known in the art. In one embodiment, the fractionation method is selected from solvent fractionation of cream, AMF, butter milk, butter serum, or beta serum, using ethanol, water, or acetone, alone or sequentially.

Lipids present in the compositions of the invention may be fully or partially modified, whether naturally, chemically, enzymatically, or by any other methods known in the art, including, for example, glycosylated, sialylated, esterified, phosphorylated or hydrolysed. Lipid hydrolysates may be prepared using known techniques, including but not limited to acid hydrolysis, base hydrolysis, enzymatic hydrolysis using a lipase, for example as described in Fox and McSweeney ((2006), Chapter 15 by HC Deeth and CH Fitz-Gerald), and microbial fermentation. One method of base hydrolysis includes adding 1% KOH (in ethanol) and heating for 10 minutes. Hydrolysed material may be neutralised with acetic acid or hydrochloric acid.

Milk fat globule membrane material may be isolated according to the acidification method of Kanno & Dong-Hyun, 1990, and further fractionated into lipid and protein fractions by the addition of methanol, as described by Kanno et al, 1975. A phospholipid extract may be isolated by extracting the lipid mixture with acetone according to the procedure of Purthi et al, 1970. Lipid residue may be further enriched in milk fat globule membrane lipids by the selective extraction of non-polar lipids with pentane.

Fractionation methods useful to produce milk fat extracts useful herein are also described in published international patent applications WO 2006/041316, WO 2007/123424, and WO 2007/123425 that are each hereby incorporated herein by reference in their entirety.

Particularly preferred milk fat extracts useful herein includes those described in the examples below and those summarised in the following Tables 1a and 1b. These extracts may be dried, and may be powders, optionally with components including flow aids such as lactose added to improve flowability. Fraction 1 is beta-serum. Fractions 2, 3, 4, and 6 are prepared by ethanol extraction of beta-serum powder. Beta serum is the liquid phase produced during AMF manufacture. The fractions including beta-serum, the G600™ milk fat precursor (Batch 1 is an emulsion, Batch 2 and Batch 3 are freeze dried powders; all manufacturing precursors to the G600™ milk fat extract), the G500™ milk fat extract, and the G600™ milk fat extract were obtained from Fonterra Co-operative Group Limited, New Zealand. Fractions 7 to 11 described in Table 2b below may be produced according to the methods described in published international patent application WO 2006/041316 (see examples 3 to 6). Fraction 11 may be produced by supercritical carbon dioxide extraction of Fraction 9.

**Table 2a - Milk fat extracts useful herein**

| | **Fraction** | | | | | |
|---|---|---|---|---|---|---|
| | **1** | **2** | **3** | **4 5 6** | | |
| **Component (%w/w)** | **Beta serum** | **G600™ precursor (Batch 1)** | **G600™ precursor (Batch 2)** | **G600™ precursor (Batch 3)** | **G500™ extract** | **G600™ extract** |
| Protein | 30.2 | ND | 10 | 7.3 | <2% | 10.2 |
| MFGM | 7.5 | ND | ND | ND | ND | ND |
| Fat | 20.6 | ND | 73 | 80 | 35.5 | 27.9 |
| Phospholipid | 9.7 | 27.6 | 44 | 46 | 17.6 | 15.1 |
| PC | 2.5 | 3.2 | 5.8 | 5.9 | 3.1 | 2.0 |
| PI | 0.8 | 6.0 | 8.4 | 8.6 | 2.8 | 2.9 |
| PS | 1.1 | 7.3 | 11.6 | 12.4 | 3.5 | 4.0 |
| PE | 2.8 | 6.4 | 12.7 | 12.4 | 4.9 | 4.4 |
| SM | 2.4 | 3.5 | 4.6 | 6.3 | 2.8 | 1.6 |
| Gangliosides | 0.4 | 4.5 | 5.8 | 5.8 | 1.3 | 2.0 |
| GD3 | 0.4 | 4.0 | 5.2 | 5.2 | 0.6 | 1.8 |
| Lactose | ND | 8.3 | 14 | 3 | 54.9 | 58.0 |
| Ash | ND | 7.0 | 10 | 7.7 | 5.0 | 8.3 |
| Moisture | 1.9 | 3.7 | 3 | 2 | 3.2 | 2.8 |

| | | | | | | |
|---|---|---|---|---|---|---|
| ND = not determined; % w/w = % by weight. | | | | | | |

**Table 2b - Milk fat extracts useful herein**

| | **Fraction** | | | | |
|---|---|---|---|---|---|
| **Component (%w/w)** | **7** | **8** | **9** | **10** | **11** |
| Protein | 49.7 | 60.2 | <0.01 | <0.01 | 12.4 |
| MFGM | 11.9 | 14.4 | 0.2 | ND | ND |
| Fat | 35.6 | 23.1 | 94.2 | 86.8 | 90.2 |
| Phospholipid | 14.9 | 16.0 | 31.0 | 65.7 | 66.8 |
| PC | 3.8 | 4.9 | 8.1 | 16.8 | 15.0 |
| PI | 1.1 | 1.5 | 2.8 | 5.8 | 6.0 |
| PS | 1.6 | 2.1 | 4.3 | 8.7 | 7.6 |
| PE | 4.3 | 5.4 | 11.3 | 23.6 | 21.8 |
| SM | 3.6 | 4.5 | 7.5 | 16.5 | 13.6 |
| Gangliosides | 0.7 | 1.0 | 1.2 | 2.0 | 2.0 |
| GD3 | 0.6 | 0.9 | 1.1 | 1.8 | 1.8 |
| Lactose | 7.8 | 11.7 | 2.6 | 6.4 | 4.0 |
| Ash | 5.2 | 5.9 | 3.1 | 12.1 | 9.1 |
| Moisture | 2.7 | 2.9 | 2.6 | 4.6 | 2.3 |

| | | | | | |
|---|---|---|---|---|---|
| ND = not determined; <0.01 = trace amounts. | | | | | |

The G500™ milk fat extract is a spray dried milk ganglioside concentrate to which lactose has been added to improve powder flowability. The G500™ milk fat extract has a typical fatty acid composition of myristic acid (14:0) 5.6%, palmitic acid (16:0) 18.4%, palmitoleic acid (16:1) 1.2%, margaric acid (17:0) 0.5%, stearic acid (18:0) 14.9%, oleic acid (18:1) 31.0%, linoleic acid (18:2) 3.8%, linolenic acid (18:3) 1.5%, and arachidonic acid (20:4) 0.5%. The G600™ milk fat extract is a spray dried milk ganglioside concentrate to which lactose has been added to improve powder flowability. The G600™ milk fat extract has a typical fatty acid composition of myristic acid (14:0) 4.7%, palmitic acid (16:0) 16.4%, palmitoleic acid (16:1) 1.2%, margaric acid (17:0) 0.5%, stearic acid (18:0) 17.0%, oleic acid (18:1) 33.4%, linoleic acid (18:2) 4.2%, linolenic acid (18:3) 1.4%, and arachidonic acid (20:4) 0.6%. Before addition of lactose, the G500™ milk fat extract and the G600™ milk fat extract are useable as precursors, with or without drying such as freeze-drying or spray-drying and without added lactose.

In the fractions described above, protein levels were determined by total nitrogen multiplied by 6.38. Phospholipid levels were determined by ³¹P NMR. Ganglioside levels were determined as follows. In triplicate, approximately 0.1g of powder was weighed into a 16 ml kimax tube and the weight recorded. 6ml of methanol was added and mixed by vortexing for 1 mm. The solution was incubated at 50°C for 10 min then 6 ml water was added and mixed by vortexing. The solution was allowed to stand for 2 hrs at 4°C to settle and a sample was taken and passed through a 0.45µm filter. The sample was analysed by HPLC. A Cosmosil™ 5NH2-MS waters column (Nacalai Tesque Inc, USA) was used with a NH2 security guard (Phenomenex™ AJO-4302 in a Phenomenex™ KJO-4282 holder). The guard cartridge was changed every day of analysis. Injections of sample were injected onto the column and eluted at a flow rate of 2ml/min using solvent A (90% acetonitrile, 5% water and 5% 5mM phosphate buffer pH5.6) and solvent B (50% acetonitrile, 45% water and 5% 200mM phosphate buffer pH5.6). The following Gradient was used: 100% A for 3.5 min, then 100% A to 55% A over 26.5 min, then 55% A to 100% A over 1 min and then 100% A for 5 min (Wagener et al. (1996), Journal of Lipid Research 37, 1823-1829). An external standard curve of 0-2 ug GD3 was generated using buttermilk GD3 (Matreya #1504). Elution was monitored at 203nm.

### 7. Compositions useful according to the invention

A composition useful herein may be formulated as a food, drink, food additive, drink additive, dietary supplement, nutritional product, medical food, enteral or parenteral feeding product, meal replacement, cosmeceutical or pharmaceutical. Appropriate formulations may be prepared by an art skilled worker with regard to that skill and the teaching of this specification.

As will be appreciated, the dose of the composition administered, the period of administration, and the general administration regime may differ between subjects depending on such variables as the mode of administration chosen, and the age, sex and/or general health of a subject.

In one embodiment, compositions useful herein include maternal formulas, infant formulas, follow-on formulas and growing up formulas, in liquid (concentrate or ready-to-drink) or powder form. Such products are formulated to target nutrients to the foetus, infant and child. It is appreciated by the first life-stages (foetus, infant and growing child) involve significant growth and development. Any support which enhances development can have significant effects on the development of the individual.

In another embodiment, compositions useful herein include dietetic products.

Examples of formulas such as maternal formula, infant formula, follow-on formula, or growing-up formula, in powder or liquid form, include the following. One example of an infant formula, follow-on formula or growing-up formula useful herein comprises (w/w)
(a) 30 - 60 % lactose
(b) 15 - 35% vegetable oils
(c) 0 - 40% skim milk powder
(d) 0 - 40% whey protein, such as a WPC or WPI, preferably an 80% WPC (WPC80), and
(e) 1 - 50% of one or more complex lipids useful herein.

Another example of an infant formula, follow-on formula or growing-up formula useful herein comprises (w/w)
(a) 40 - 60 % lactose
(b) 20 - 30% vegetable oils
(c) 10 - 15% skim milk powder
(d) 6 - 8% whey protein, preferably WPC80, and
(e) 1 - 10% of one or more complex lipids useful herein.

One example of a material formula useful herein comprises (w/w)
(a) 80-99.9% of a milk powder selected from whole milk powder, skim milk powder, milk protein concentrate (MPC), milk protein isolate (MPI), and whey protein such as a WPC or WPI
(b) 0-20% lipid such as milk fat or one or more vegetable oil
(c) 0-25% sugars or carbohydrate ingredient
(d) 0.1-0.5% vitamin and mineral mix
(e) 0-5% flavour ingredients, and
(f) 0-5% one or more complex lipids useful herein.

Any of these formulas may also comprise 0.1 to 4% w/w, preferably 2 to 4% w/w/ of one or more of a vitamin premix, a mineral premix, lecithin, one or more antioxidants, one or more stabilisers, or one or more nucleotides, or a combination of any two or more thereof. In some embodiments, these formulas may be formulated to provide from about 2700 to about 3000 kJ/L.

In alternative embodiments, the compositions useful herein may be formulated to allow for administration to a subject by any chosen route, including but not limited to oral administration.

The following non-limiting examples are provided to illustrate the present invention and in no way limits the scope thereof.

### EXAMPLES

### Test Material

G600™ precursor (Fonterra Co-operative Group Limited) is a dairy derived complex lipid ingredient having the composition shown in the following table (energy 2730 kJ/100 g).

**Table 3 - G600™ precursor composition**

| **Component** | **% w/w** |
|---|---|
| Protein | 7.3 |
| Fat | 80 |
| Phospholipid | 46 |
| PC | 5.9 |
| PI | 8.6 |
| PS | 12.4 |
| PE | 12.4 |
| SM | 6.3 |
| Gangliosides | 5.8 |
| GD3 | 5.2 |
| Docosahexaenoic acid (DHA) | 0.034 |
| Choline | 0.45 |
| Lactose | 3 |
| Ash | 7.7 |
| Moisture | 2 |

The measurement of gangliosides in the G600™ precursor was conducted by dissolving 0.1 g in 12.5 mL of methanol followed by incubation at 50°C for 10 minutes followed by the addition of 12.5 mL of water and incubation at 4°C for 2 hours then analysis by HPLC-UV (Wagner et al., 1996).

### EXAMPLE 1 - Effect on cognition and learning

### 1.1 Timed-matings

Timed mating of Wistar rats was performed at 100 days of age using estrous cycle monitor (EC-40, Fine Science Tools, San Francisco, USA). Confirmation of mating was via a vaginal lavage with sterile saline and visualisation of spermatozoa under a microscope. Pregnancy success rate was 100% with 28 dams mated.

### 1.2 Nutritional supplementation

Dams were fed a standard rat chow (Diet 2018, Harlan Teklad, Oxon, UK) throughout pregnancy and lactation that contained no complex milk lipids. Dams were supplemented with gangliosides using the gel described below.

Neonates were supplemented with gangliosides from postnatal day 10 until day 22 (early post-weaning) at a concentration of 0.02% (low) to 1% (high) weight/bodyweight (w/w) relative to measured food intake.

Gels for dams and pups were prepared using a gel formulation comprising 10% w/v gelatin, 10% w/v sucrose, 5% v/v flavouring concentrate (Hansells™ Raspberry flavouring) and 0 (Blank), 0.384% w/v (Low) or 1.92% w/v (High) of G600™ precursor. Equivalent to 0, 48 and 240 mg per 12.5ml gel per day.

### 1.3 Pre-weaning supplementation

The supplement was administered by oral gavage using feeding tubes specifically designed for use in rat pups/weanlings (Instech Laboratories, product number FTP 20-30, 20 gauge, (9 mm OD x 0.5 mm ID) x 30 mm)). Dose was 0.1 ml in sterile water. Control animals were administered a sham gavage containing sterile water.

### 1.4 Post-weaning gel supplementation

At weaning (postnatal day 22), animals were weight-matched within treatment group and housed two per cage under standard conditions. The chow diet (described above) was fed to pups and was supplemented with a ganglioside-containing gel described above at the doses stated above.

The chow diet was supplemented with the gels at the doses defined above based on food intake and adjusted according to changes in dietary consumption. The gels were placed at either end of the feeding platform in each cage and the gels were preferentially consumed over the chow. There were no significant gel remnants from any of the animals using this technique.

### 1.5 Morris Water Maze Testing

The Morris Water Maze is widely used in the Wistar rat. The Morris Water Maze is based upon the premise that animals have evolved an optimal strategy to explore their environment and escape from the water with a minimum amount of effort. The protocol was taken from Current Protocols in Neuroscience (Vol 3, Section 8.5A.5) and was performed over a 5 day period. In its most basic form, the water maze assesses spatial learning and memory (Brandeis et al, 1989). All data was collected automatically via computer using an automated tracking system (AnyMaze, Stoelting, USA).

### 1.5.1 Morris Water Maze Testing Results

Figure 1 shows the time each group of rats spent to reach the submerged platform during 4 days of acquisition testing. All groups were similar at the start of acquisition testing. The effect of learning over time was *p*<0.0001 for all groups. On day 2, the High dose gel treated animals learnt significantly (p<0.05) quicker than the Blank gel and Low dose gel treatment groups. However, by day 4, all groups were similar in their ability to locate the platform.

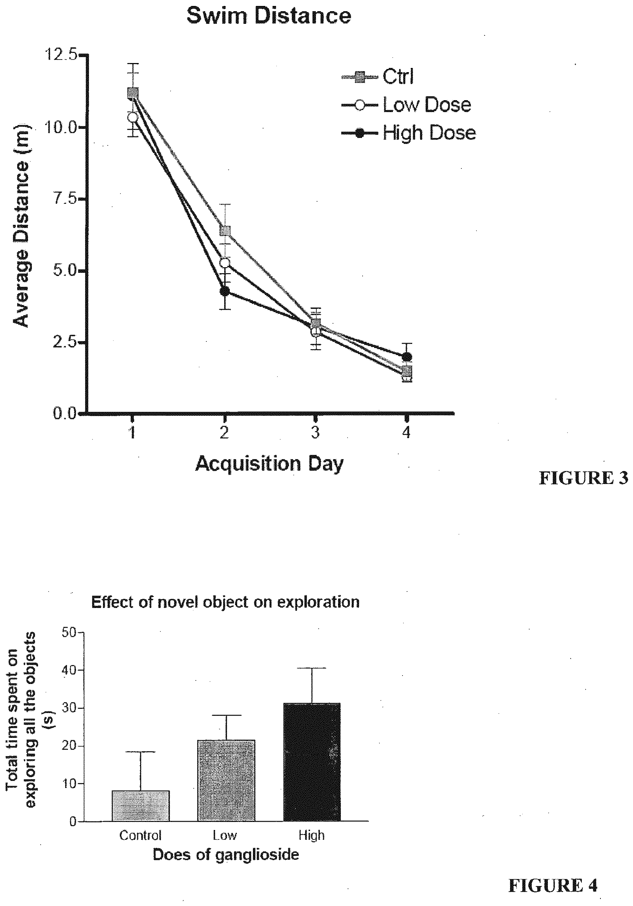

Figure 2 shows the average swim speed over the 4 days of acquisition testing in the water maze and Figure 3 shows the average swim distance. There were no significant differences in swim speed between any of the treatment groups. The trend for change in swim speed over time was p<0.0001 for all treatment groups. There was a trend towards decreased swim speed in High dose gel treated animals (p=0.09). With swim distance, there was a significant difference in overall swim length in High dose gel treated animals versus Blank gel (controls) on day 2 of testing which parallels the shortened time to platform in this group (*p*<0.05). The trend for change in swim distance over time was *p*<0.0001 for all treatment groups.

### 1.5.2 Morris Water Maze Summary

There was an improved rate of learning and swim parameters of distance and speed on day 2 of the acquisition phase in High dose gel treated animals. But by day four there where no significant differences between the groups. The results indicated the High dose animals learnt the task faster than the control animals. The analysis indicates the animals did this by shorter swim distance and without swimming faster. Conversely no detrimental effects of G600™ precursor were observed on the parameters tested. Overall therefore the data indicate that G600™ precursor is supportive of learning.

### 1.6 Novel Object Recognition Task Testing

The Novel Object Recognition task if also widely used in the Wistar rat. Exploration is a typical learning behaviour for rats when they have been placed into a novel environment. (Ennaceur and Delacour, 1988)

### 1.6.1 Novel Object Recognition Task Results

Total exploratory activity, measured by the time spent on exploring 4 different objects, gradually reduced from approximately 120 second to 80 seconds consistent with the inventors experience with this model. The learning slopes were similar between the groups (data not shown). Notably, increases in exploring activity were more prominent in the groups treated with Low dose gel or High dose gel compared to the Blank gel treated group when a novel object was introduced during the second trial of testing day 4.

Following 1 of 4 familiar objects being replaced with a novel object, there was an approximate 3-4 fold increase in exploratory activity in the groups treated with Low dose gel or High dose gel, particularly the High dose group (31.14 ± 9.34) compared to the Blank (control) treated group (8.28 ± 10.22, p=0.011, two tailed t-test). These data may suggest that the treatment with complex lipids improved the awareness of an altered environment (Figure 4).

### 1.6.2 Novel Object Recognition Task Summary

The introduction of a novel object did result in a significant (*p*=0.011) 3-4 fold increase in exploring activity in the groups treated with G600™ precursor. These data may suggest more awareness of changing environment by complex lipid supplementation. Conversely no detrimental effects of supplementation were observed in this system.

### EXAMPLE 2 - Effect on growth

### 2.1 Timed-matings and nutritional supplementation

Timed mating of Wistar rats and nutritional supplementation was performed as described for Example 1.

### 2.2 DEXA scanning

Body composition was assessed using dual energy X-ray absorptiometry (DEXA). The DEXA instrument differentiates body weight into the components of lean soft tissue, fat soft tissue and bone, based on the differential attenuation by tissues of two levels of x-rays. This technique allows determination of whether growth includes or is independent of an increase in body fat composition or bone mineral/density.

### 2.3 Results

Twenty eight dams were time mated using and estrous cycle monitor and all 28 pregnancies were successful. The litter from one dam was excluded from the experimental study due to small litter size and macrosomic pups. Litter size, pup weight and male:female sex ratios were all well within the normal range (mean litter size 14 ± 2, mean pup weight 6.2 ± 0.2 g). Litters were adjusted to 8 pups to standardise nutrition until weaning. Males were used with the balance made up with females where necessary.

At neonatal day 10, animals were randomly assigned within litter to receive a Blank, Low dose or High dose by gavage as detailed in Example 1 above. For example in a litter containing 6 males and 2 females, 2 males were treated per treatment group and females left untreated. All treated pups were handled identically. No mortalities or adverse events were observed in any of the animals (n=156 pups dosed). Neonatal dosing by gavage ceased at weaning (day 22) and replaced with oral supplementation with gels as described above.

Weaning weights were in the normal range although there was a small but significant increase (p<0.05) in weaning weight in the High dose gel treated animals compared to controls: Blank gel 61.49 ± 0.43 g, Low dose gel 62.33 ± 0.42 g, High dose gel 63.22 ± 0.48 g.

### 2.3.1 Postnatal Growth

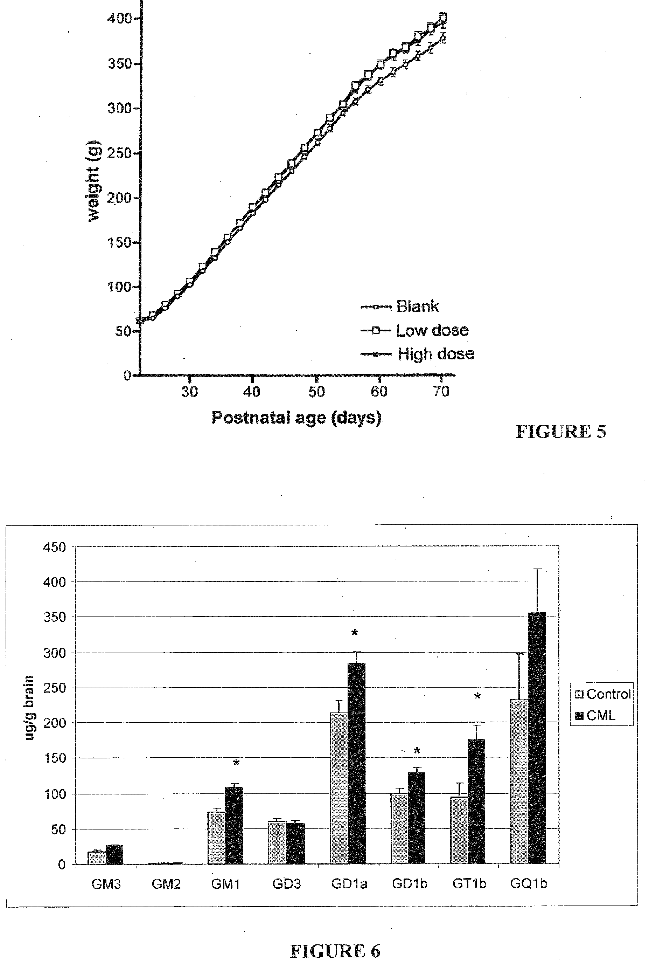

Postnatal growth was significantly increased in offspring supplemented with G600™ precursor (either Low dose gel or High dose gel). This is shown in Figure 5. This effect was not dose dependent and is also independent of the marginal calorific effects of the gel supplement. By weaning (day 22) there was a small but significant increase in body weight in the High dose group compared to control. By postnatal day 80 there was a significant increase in body weight in both treatment groups (p<0.05).

### 2.3.2 Body composition

There were no significant differences in body fat composition or bone mineral/density markers between any of the treatment groups. Thus, the increased weight gain observed in animals supplemented with G600™ precursor (either Low dose gel or High dose gel) did not reflect increased adiposity. There was a slight trend towards an increased fat:lean ratio in Low dose gel treated animals compared to controls (p=0.1). High dose gel treated animals were slightly but significantly longer (p<0.05, nose-anus length) than control animals. These results are shown in Table 4 below. There was a small but significant increase in BMD in High dose treated animals compared to controls (p<0.05).

**Table 4 - Total body fat, fat:lean ratios, bone mineral density and body lengths. Data are mean ± SEM, n=16 per group.**

| **Group** | **% Fat** | **Fat:lean ratio** | **BMD** | **Length (mm)** |
|---|---|---|---|---|
| **Control** | 20.9 ± 0.7 | 0.266 ± 0.01 | 0.147 ± 0.001 | 245.3 ± 1.5 |
| **Low dose gel** | 22.2 ± 0.8 | 0.297 ± 0.02** | 0.148 ± 0.001 | 247.5 ± 1.56 |
| **High dose gel** | 21.1 ± 0.8 | 0.270 ± 0.01 | 0.150 ± 0.001* | 249.7 ± 1.38* |

| | | | | |
|---|---|---|---|---|
| *: p<0.05 compared to control; **: p<0.1 compared to control | | | | |

### 2.4 Finding

The inventors therefore discovered that complex lipid supplementation led to an unexpected but significant increase in body weight gain (both Low dose and High dose gel groups) and length (High dose group). This growth was not due to increased adiposity as assessed by DEXA scanning.

### EXAMPLE 3 - Placental transfer trial

Dual perfused human lobules were taken from six placentae of uncomplicated term pregnancies (normal elective Caesarean patients). Maternal and fetal arterial and venous supplies were isolated, catheterised and the placenta maintained by perfusion of sterile krebs buffer (that also contains serum albumin to aid transport of lipophilic substances) at room temperature in a sterile air perfusion chamber at constant flow rate and pressure. The perfusion technique used was modified from Glance et al (1984) according to Collier et al (2004).

Equilibration was performed for 60 min to assess physical integrity of tissue and circuits (fetal arterial pressure < 40 mmHg and leakage of perfusate from fetal to maternal circuit < 2 ml/h) then the maternal and fetal reservoirs replaced with fresh media containing doses of test substances. Time zero and then hourly samples are collected from all circuits and stored frozen for analysis. Metabolic viability of the preparation is assessed with samples collected hourly from maternal and fetal arteries and analysed for oxygen and carbon dioxide saturation, electrolytes and glucose consumption (Bayer 865 Blood Gas Analyzer, Bayer Diagnostics and lactate production (Hitachi 917, Roche Diagnostics). 10 µg/mL of milk-derived ganglioside mixture (GM3 + GD3 = isolated from G600™ precursor described above) was added to the maternal reservoir. Perfusion was conducted for up to 3 hours using a closed loop system. Samples were taken from both reservoirs at that point (t=0) and at regular time-points, snap frozen in liquid nitrogen, and subsequently assayed for GM3, GD3, and lobule viability.

GM3 and GD3 contents of the perfusates were measured by LC-MS using a Hypersil APS2 column, normal phase acetonitrile gradient and detected in the LTQ orbitrap in negative ion full scan, as described below. Perfusate samples were "protein crashed" by addition into methanol, allowed to stand for 60 min at 4°C before centrifugation (20,000 x g). An aliquot (10-50 µL) was loaded onto an online C18 trap, washed (50% MeOH), before eluted onto a Hypersil APS2 column (Thermo Finnigan, MA, USA). Gangliosides were separated on a normal phase gradient, and detected in the LTQ Orbitrap Mass spectrometer (ThermoFinnigan, Ma, USA) in negative ion mode. The LC-MS system was calibrated using GD3 and GM3 standards purchased from Matreya (Pa, USA). Lobule viability was assessed by Glucose uptake and lactate production, measured with an Autoanalyzer.

The inventors discovered that in all experiments there was evidence of transfer of GM3. GM3 foetal perfusate concentrations increased approximately 6-fold over time and by a significant amount over background. Maternal perfusate GM3 concentrations were reduced on average by half from approximately 300 ng/ml. The inventors also discovered that there was evidence in all cases of a significant reduction in maternal GD3 concentrations averaging 25-30% but the concentrations in foetal perfusate were below assay sensitivity.

The inventors therefore conclude that gangliosides can transfer across the human placenta. Although uptake of both GD3 and GM3 from the maternal perfusate occurs, there appears to be a preference for GM3 uptake and release into the foetal side.

### EXAMPLE 4 - Assessment of brain gangliosides

An HPLC-MS method was used to measure changes in the relative concentration of 8 classes of brain gangliosides (GM1, GM2, GM3, GD3, GD1a, GD1b, GT1b, and GQ1b). The acquired mass spectra were filtered post analysis for the known masses of each ganglioside species. The method was then used to compare the ganglioside levels in the brains of 2 day old rat pups.

Pregnant rats were fed a diet supplemented with either a dairy derived complex lipid (G600™ precursor, described above) or a calorific equivalent control. Two days after birth the pups were sacrificed and the brain lipids extracted using the extraction protocol reported by Svennerholm and Fredman (1980). The Svennerholm & Fredman method was scaled down for rat brain extraction using initial solvent proportions of 2 mL water (including 0.25g sample), 5.4 mL CH₃OH, and 2.7 mL CHCl₃. The final upper phase extract containing gangliosides were made up to a final volume of 20 mL in CH₃OH: water (50:50). Chloroform containing ethanol as a stabiliser was used.

The upper phase was used for ganglioside LC-MS analysis. HPLC analysis was performed on an Agilent 1100 series HPLC system consisting of a quaternary pump, binary pump, degasser, column heater (60°C) and refrigerated auto-sampler (5°C). Samples or standards (10uL) were initially loaded (0.5 mL/min; 50% MeOH) onto a wide pore, reversed-phase trap (C18, 4 x 2.0 mm, 5 u, Phenomenex, CA, USA) for preconcentration/desalting. After 2 min, the trap was switched in-line with an APS-2 Hypersil hydrophilic column (150 mm x 2.1 mm, 3 µm, Thermo) coupled to an APS-2 guard column (10 mm x 2.1 mm id). The gangliosides were separated with an acetonitrile (ACN)/ ammonium acetate buffer gradient described in Table 5 where Solvent A comprised 95% Acetonitrile, 5% 50 mM Ammonium Acetate Buffer, pH 5.6, Solvent B comprised 50:50 acetonitrile : 50mM Ammonium Acetate buffer, pH 5.6, and Solvent C comprised. 50:50 MeOH: Water. The flowrate: 0.5 mL/mm

**Table 5 - HPLC gradient**

| Time (min) | Trap valve | 96A | 96B | 96C |
|---|---|---|---|---|
| 0 | | 95 | 5 | 0 |
| 2 | Divert to waste | 95 | 5 | 0 |
| 4 | | 95 | 5 | 0 |
| 8 | Dive rt to detector | | | |
| 15 | | 10 | 90 | 0 |
| 16 | | 0 | 0 | 100 |
| 18 | | 0 | 0 | 100 |
| 19 | Divert to waste | 95 | 5 | 0 |
| 25 | | 95 | 5 | 0 |

Negative polarity electrospray ionisation, in full scan mode (700-1650 m/z), at a resolution of 30000 was used for the analysis of the 8 ganglioside classes named above. The acquired spectra were filtered post analysis for the known masses of each ganglioside species. Analysis time was 25 minutes.

Ganglioside standards (Matreya, Pleasant Gap, PA, USA), were prepared at 1 mg/mL or 0.5mg/mL in methanol: water (50:50), and serially diluted to give an 7 point standard curve for each ganglioside class that was used for comparison of the similarities and differences in composition and relative amount of these ganglioside classes found in the extracts of the control and treatment groups (Brugger et al, 1997; Koivusalo M et al, 2001).

Total ganglioside was significantly increased in the treatment group (p=0.013 compared to control). While the distribution of the major brain gangliosides was not significantly different, amounts of GM1, GD1a, GD1b, and GTlb were significantly increased (Figure 6; p<0.05). Brain weight for the treatment group was also significantly higher (p=0.003).

### INDUSTRIAL APPLICATION

The present invention has utility in achieving particular health benefits including maintaining or increasing cognitive development and maintaining or increasing growth. The described formulations and compositions may be employed in a number of different ways including maternal formulas, infant formulas, follow-on formulas, growing-up formulas or dietetic products.

Those persons skilled in the art will understand that the above description is provided by way of illustration only and that the invention is not limited thereto.

### REFERENCES

Astaire J. C., Ward R., German J. B., and Jimenez-Flores R. (2003) Concentration of Polar MFGM Lipids from Buttermilk by Microfiltration and Supercritical Fluid Extraction J. Dairy Sci. 86, 2297-2307
Bode, L., C. Beermann, et al. (2004). Human and bovine milk gangliosides differ in their fatty acid composition. Journal of Nutrition 134(11): 3016-3020.
Bremer, E.G., Hakomori, S., Bowen-Pope, D.F., Raines, E., Ross, R. (1984) Ganglioside-mediated Modulation of Cell Growth, Growth Factor Binding, and Receptor Phosphorylation The Journal of Biological Chemistry, 259(11): 6818-6825.
Brandeis et al 1989, The use of the Morris Water Maze in the study of memory and learning, Int J NeuroSci 48; 29-69
Brugger B, Erben G, Sandhoff R, Wieland F T & Lehmann W D (1997) Quantitative analysis of biological membrane lipids at the low picomole level by nano-electrospray ionisation tandem mass spectrometry. Proc. Natl. Acad. Sci, 94, 2339-2344. Bryan J, Osendarp S, Hughes D, et al (2004). Nutrients for Cognitive development in school-aged children. Nutr Rev 62: 295-306.
Bylund, G. (Ed.) Dairy processing handbook. (1995) Tetra Pak Processing Systems AB, S-221 86 Lund, Sweden.
Collier, A.C. et al., (2004), Human Placental Glucuronidation and Transport of 3'-Azido-3'-Deoxythymidine and Uridine Diphosphate Glucuronic Acid. Drug Metabolism and Disposition. 32 (8), 813-820.
Ennaceur A, Delacour J. A new one-trial test for neurobiological studies of memory in rats. 1: Behavioral data. Behav Brain Res. 1988 Nov 1;31(1):47-59.
Fox PF, McSweeney PLH (eds), Advanced Dairy Chemistry, Volume 2 - Lipids, 3rd Ed, Springer Science + Business Media, Inc., 2006).
Freireich EJ, Gehan EA, Rail DP, Schmidt LH, Skipper HE (1966) Quantitative comparison of toxicity to anticancer agents in mouse, rat, hamster, dog, monkey and man. Cancer Chemother Rep 50: 219-244.
Glance D.G. et al., (1984), The effects of the components of the renin-angiotensin system on the isolated perfused human placental cotyledon. American Journal of Obstetrics & Gynecology. 149 (4), 450-454.
Hungund B.L. et al., (1993), Placental transfer of (3H)-GM1 and its distribution to maternal and fetal tissues of the rat. Life Sciences. 53, 113-119.
Illingworth, D., Fractionation of fats. In Physical Properties of Lipids (Marangoni A G & Narine S S, Eds), pp. 411-448. Marcel Dekker, New York (2002).
Kanno C & Dong-Hyun K (1990). A simple procedure for the preparation of bovine milk fat globule membrane and a comparison of its composition, enzymatic activity, and electrophoretic properties with these prepared by other methods. Agric. Biol. Chem., 54(11):2845-2854.
Kanno C, Shimizu M & Yamachi K (1975). Isolation and physiochemical properties of a soluble glycoprotein fraction of milk fat globule membrane. Agric. Biol. Chem., 39(9):1835-1842.
Koivusalo M, Haimi P, Heikinheimo L, Kostiainen R & Somerharju P(2001). Quantitative determination of phospholipids compositions by ESI-MS: effects of acyl chain length, unsaturation, and lipid concentration on instrument response. Journal of Lipid Research, 42, 663-672.
Martin, M. J., S. Martin-Sosa, et al. (2001). Distribution of bovine milk sialogly coconjugates during lactation. Journal of Dairy Science 84(5): 995-1000.
Mendez-Otero R & Santiago MF (2003), Functional role of a specific ganglioside in neuronal migration and neurite outgrowth, Brazilian Journal of Medical and Biological Research 36, 1003-1013
Neeser, J. R., M. Golliard, et al. (1991). Quantitative determination of complex carbohydrates in bovine milk and in milk-based infant formulas. J Dairy Sci 74(9): 2860-2871.
XL Pan, T Izumi, Variation of the ganglioside compositions of human milk, cow's milk and infant formulas, Early Hum Dev, 2000 57(1):25-31
Pruthi T D, Narayanan K M & Bhaleerao V R (1970). The role of milk phospholipids in the autoxidation of butterfat = I. Indian Journal of Dairy Science, 23:248-251
Rahmann H (1995), Brain Gangliosides and Memory Formation, Behavioural Brain Research 66, 105-116
Rombaut R, Camp JV, Dewettinck K., Analysis of phospho- and sphingolipids in dairy products by a new HPLC method. J Dairy Sci. (2005) 88(2):482-8.
Rombaut R, Dejonckheere V, Dewettinck K., Microfiltration of butter serum upon casein micelle destabilization. J Dairy Sci. (2006)(a) 89(6):1915-25.
Rombaut R., Van Camp J. & Dewettinck K., Phospho- and sphingolipid distribution during processing of milk, butter and whey, International Journal of Food Science & Technology, (2006)(b) 41(4):435-443.
H Rosner, Developmental expression and possible roles of gangliosides in brain development, Prog Mol Subcell Biol, 2003;32:49-73.
R Rueda, The role of dietary gangliosides on immunity and the prevention of infection, British Journal of Nutrition, 2007 98 Suppl 1:S68-73
Rueda, R., Gil, A. (1998). Roles. Lipids in Infant Nutrition. Y. S. Huang, and Sinclair, A.J. Champaign, IL, AOCS Press: 213-234.
Sanchez-Dial A, Ruano MJ, Lorente F, Hueso P. (1997). A critical analysis of total sialic acid and sialoglycoconjugate contents in bovine milk-based infant formulas. J Pediatric Gastroenterol Nutr, 24:405-10.
Schauer, R, Sialic acids: fascinating sugars in higher animals and man, Zoology 2004 107(1):49-64
L Svennerholm, Chromatographic Separation Of Human Brain Gangliosides, Journal of Neurochemistry, 1963, 10:613-23.
Svennerholm, L., K. Bostrom, et al. (1994). Membrane lipids of adult human brain: lipid composition of frontal and temporal lobe in subjects of age 20 to 100 years. J Neurochem 63(5): 1802-11.
Svennerholm L and Fredman P, A procedure for the quantitative isolation of brain ganglioside. Biochimica et Biophysica Acta, (1980) 617, 97-109.
TH Tram, JCB Miller, Y McNeil, P McVeagh, Sialic acid content of infant saliva: comparison of breast fed with formula fed infants, Archives of Disease in Childhood, 1997; 77(4):315-8
Wagner R., Kobbe, B., & Stoffel, W. (1996). Quantification of gangliosides by microbore high performance liquid chromatography. Journal of Lipid Research 37:1823-1829.
WHO (1981). International Code of Marketing Breastmilk Substitutes. World Health Organisation, Geneva.

The present invention will now be described by way of reference to the following clauses:
1. One or more complex lipids for maintaining or increasing growth of a foetal, infant, or child subject.
2. The product of clause 1, wherein the product is administered to a mother during gestation and the growth is brain weight of a foetal subject or brain ganglioside content of a foetal subject.
3. The product of clause 1, wherein the product is administered to an infant or child subject and the growth is one or more of body weight, body length, and bone mineral density.
4. One or more complex lipids for maintaining or increasing cognitive development in a foetal, infant, or child subject.
5. The product of clause 4, wherein the product is administered to a mother during gestation and the cognitive development is brain weight of a foetal subject or brain ganglioside content of a foetal subject.
6. The product of any one of the preceding clauses, wherein the one or more complex lipids comprises one or more phospholipids, one or more sphingolipids, one or more sphingomyelins or derivatives thereof, one or more ceramides, one or more cerebrosides, one or more gangliosides, or any combination of any two or more thereof.
7. The product of clause 6, wherein the one or more gangliosides comprises GM3, GD3, or a mixture of at least GM3 and GD3.
8. The product of any one of the preceding clauses, wherein the one or more complex lipids comprises at least about 0.1% gangliosides w/w on a dry basis.
9. The product of any one of the preceding clauses, wherein the product comprises at least about 2 mg gangliosides per 100g.
10. The product of clause 9, wherein the product comprises about 5 mg to about 20 mg gangliosides per 100g.
11. The product of any one of the preceding clauses, wherein the one or more complex lipids comprises a milk fat extract.
12. The product of clause 11 , wherein the milk fat extract comprises about 15% to about 99% by weight total lipid, about 1% to about 80% by weight phospholipid, about 1% to about 25% by weight phosphatidylcholine, about 0.1% to about 15% by weight phosphatidylinositol, about 0.1% to about 15% by weight phosphatidylserine, about 1% to about 30% by weight phosphatidylethanolamine, about 0.5% to about 25% by weight sphingomyelin, and about 0.1 to about 10% by weight ganglioside.
13. The product of clause 12, wherein the product comprises at least about 2 mg milk fat extract per 100g.
14. The product of clause 12, wherein the product comprises at least about 5 mg milk fat extract per 100g.
15. The product of clause 12, wherein the product comprises at least about 7.5 mg milk fat extract per 100g.
16. Use of one or more complex lipids in the manufacture of a formulation for maintaining or increasing growth of a foetal, infant, or child subject.
17. The use of clause 16, wherein die formulation is administered to a mother during gestation and the growdi is brain weight of a foetal subject or brain ganglioside content of a foetal subject.
18. The use of clause 16, wherein the formulation is administered to an infant or child subject and the growth is one or more of body weight, body length, and bone mineral density.
19. Use of one or more complex lipids in the manufacture of a formulation for maintaining or increasing cognitive development in a foetal, infant, or child subject.
20. The use of clause 19, wherein the formulation is administered to a mother during gestation and die cognitive development is brain weight of a foetal subject or brain ganglioside content of a foetal subject.
21. The use of any one of clauses 16 to 20, wherein the one or more complex lipids comprises one or more phospholipids, one or more sphingolipids, one or more sphingomyelins or derivatives diereof, one or more ceramides, one or more cerebrosides, one or more gangliosides, or any combination of any two or more thereof.
22. The use of clause 21, wherein the one or more gangliosides comprises GM3, GD3, or a mixture of at least GM3 and GD3.
23. The use of any one of clauses 16 to 22, wherein the one or more complex lipids comprises at least about 0.1% gangliosides w/w on a dry basis.
24. The use of any one of clauses 16 to 23, wherein the formulation comprises at least about 2 mg gangliosides per 100g.
25. The use of clause 24, wherein the formulation comprises about 5 mg to about 20 mg gangliosides per 100g.
26. The use of any one of clauses 16 to 25, wherein- the one or more complex lipids comprises a milk fat extract.
27. The use of clause 26, wherein the milk fat extract comprises about 15% to about 99% by weight total lipid, about 1% to about 80% by weight phospholipid, about 1% to about 25% by weight phosphatidylcholine, about 0.1% to about 15% by weight phosphatidylinositol, about 0.1% to about 15% by weight phosphatidylserine, about 1% to about 30% by weight phosphatidylethanolamine, about 0.5% to about 25% by weight sphingomyelin, and about 0.1 to about 10% by weight ganglioside.
28. The use of clause 27, wherein the formulation comprises at least about 2 mg milk fat extract per 100g.
29. The use of clause 27, wherein the formulation comprises at least about 5 mg milk fat extract per 100g.
30. The use of clause 27, wherein the formulation comprises at least about 7.5 mg milk fat extract per 100g.
31. A method for maintaining or increasing growth of a foetal, infant, or child, the method comprising administering a composition comprising one or more complex lipids to a foetal, infant, or child subject in need thereof.
32. The method of clause 31, wherein the composition is administered to a mother during gestation and the growth is brain weight of a foetal subject or brain ganglioside content of a foetal subject.
33. The method of clause 31, wherein the composition is administered to an infant or child subject and the growth is one or more of body weight, body length, and bone mineral density.
34. A method for maintaining or increasing cognitive development of a foetal, infant, or child subject by administering a composition comprising one or more complex lipids to a foetal, infant, or child subject in need thereof.
35. The method of clause 34, wherein the composition is administered to a mother during gestation and the cognitive development is brain weight of a foetal subject or brain ganglioside content of a foetal subject.
36. A method for maintaining or increasing growdi or maintaining or increasing cognitive development of a foetal subject, the method comprising providing a pregnant mother with a composition comprising one or more complex lipids and informing the mother that the composition will maintain or increase growth or maintain or increase cognitive development of the foetal subject.
37. A method for maintaining or increasing growth or maintaining or increasing cognitive development in a subject, the method comprising providing a subject with a composition comprising one or more complex lipids and informing the subject that the composition will maintain or increase growth or maintain or increase cognitive development of the subject.
38. The method of any one of clauses 31 to 37, wherein the one or more complex lipids comprises one or more phospholipids, one or more sphingolipids, one or more sphingomyelins or derivatives thereof, one or more ceramides, one or more cerebrosides, one or more gangliosides, or any combination of any two or more thereof.
39. The method of clause 38, wherein the one or more gangliosides comprises GM3, GD3, or a mixture of at least GM3 and GD3.
40. The method of any one of clauses 31 to 39, wherein the one or more complex lipids comprises at least about 0.1% gangliosides w/w on a dry basis.
41. The method of any one of clauses 31 to 40, wherein the composition comprises at least about 2 mg gangliosides per 100g.
42. The method of clause 41, wherein the composition comprises about 5 mg to about 20 mg gangliosides per 100g.
43. The method of any one of clauses 31 to 42, wherein die one or more complex lipids comprises a milk fat extract.
44. The method of clause 43, wherein the milk fat extract comprises about 15% to about 99% by weight total lipid, about 1% to about 80% by weight phospholipid, about 1% to about 25% by weight phosphatidylcholine, about 0.1% to about 15% by weight phosphatidylinositol, about 0.1% to about 15% by weight phosphatidylserine, about 1 % to about 30% by weight phosphatidylethanolamine, about 0.5% to about 25% by weight sphingomyelin, and about 0.1 to about 10% by weight ganglioside.
45. The method of clause 44, wherein the composition comprises at least about 2 mg milk fat extract per 100g.
46. The method of clause 44, wherein the composition comprises at least about 5 mg milk fat extract per 100g.
47. The method of clause 44, wherein the composition comprises at least about 7.5 mg milk fat extract per 100g.

## Claims

1. One or more complex lipids for maintaining or increasing growth of a foetal, infant, or child subject, wherein the one or more complex lipids comprises at least about 0.1% gangliosides w/w on a dry basis.

2. The product of claim 1, wherein the product is administered to a mother during gestation and the growth is brain weight of a foetal subject or brain ganglioside content of a foetal subject.

3. The product of claim 1, wherein the product is administered to an infant or child subject and the growth is one or more of body weight, body length, and bone mineral density.

4. One or more complex lipids for maintaining or increasing cognitive development in a foetal, infant, or child subject, wherein the one or more complex lipids comprises at least about 0.1% gangliosides w/w on a dry basis.

5. The product of claim 4, wherein the product is administered to a mother during gestation and the cognitive development is brain weight of a foetal subject or brain ganglioside content of a foetal subject.

6. The product of any one of the preceding claims, wherein the one or more complex lipids comprises one or more phospholipids, one or more sphingolipids, one or more sphingomyelins or derivatives thereof, one or more ceramides, one or more cerebrosides, one or more gangliosides, or any combination of any two or more thereof.

7. The product of claim 6, wherein the one or more gangliosides comprises GM3, GD3, or a mixture of at least GM3 and GD3.

8. The product of any one of the preceding claims, wherein the product comprises at least about 2 mg gangliosides per 100g or about 5 mg to about 20 mg gangliosides per 100g.

9. The product of any one of the preceding claims, wherein the one or more complex lipids comprises a milk fat extract.

10. The product of claim 9, wherein the milk fat extract comprises about 15% to about 99% by weight total lipid, about 1% to about 80% by weight phospholipid, about 1% to about 25% by weight phosphatidylcholine, about 0.1% to about 15% by weight phosphatidylinositol, about 0.1% to about 15% by weight phosphatidylserine, about 1% to about 30% by weight phosphatidylethanolamine, about 0.5% to about 25% by weight sphingomyelin, and about 0.1 to about 10% by weight ganglioside.

11. The product of claim 10, wherein the product comprises at least about 2 mg milk fat extract per 100g, at least about 5 mg milk fat extract per 100g, or at least about 7.5 mg milk fat extract per 100g.

12. The product of any one of the preceding claims, wherein the one or more complex lipids comprises
(a) about 15 to about 25% w/w lipid, about 5 to about 15% w/w phospholipid, and about 0.1 to about 1% w/w ganglioside, or
(b) about 15 to about 25% w/w lipid, about 5 to about 15% w/w phospholipid, about 1 to about 5% w/w phosphatidylcholine, about 0.1 to 2% w/w phosphatidylinositol, about 0.5 to about 2% w/w phosphatidylserine, about 1.5 to about 6% w/w phosphatidylethanolamine, about 1 to about 5% w/w sphingomyelin, and about 0.1 to about 1% w/w ganglioside, or
(c) about 25 to about 45% w/w lipid, about 10 to about 25% w/w phospholipid, and about 0.1 to about 2.0% w/w ganglioside, or
(d) about 25 to about 45% w/w lipid, about 10 to about 25% w/w phospholipid, about 1 to about 5% w/w phosphatidylcholine, about 0.1 to 2% w/w phosphatidylinositol, about 0.5 to about 2% w/w phosphatidylserine, about 1.5 to about 6% w/w phosphatidylethanolamine, about 1 to about 5% w/w sphingomyelin, and about 0.1 to about 2.0% w/w ganglioside, or
(e) about 12 to about 32% w/w lipid, about 5 to about 25% w/w phospholipid, and about 0.1 to about 2.0% w/w ganglioside, or
(f) about 12 to about 32% w/w lipid, about 5 to about 25% w/w phospholipid, about 2 to about 8 % w/w phosphatidylcholine, about 0.5 to 3% w/w phosphatidylinositol, about 1 to about 3.5% w/w phosphatidylserine, about 1 to about 10% w/w phosphatidylethanolamine, about 1 to about 8% w/w sphingomyelin, and about 0.5 to about 2.5% w/w ganglioside, or
(g) about 80 to about 99% w/w lipid, about 20 to about 75% w/w phospholipid, and about 0.5 to about 5% w/w ganglioside, or
(h) about 80 to about 99% w/w lipid, about 20 to about 75% w/w phospholipid, about 2 to about 22 % w/w phosphatidylcholine, about 1 to about 10% w/w phosphatidylinositol, about 1 to about 10% w/w phosphatidylserine, about 5 to about 30% w/w phosphatidylethanolamine, about 1 to about 20% w/w sphingomyelin, and about 0.5 to about 5% w/w ganglioside, or
(i) about 90 to about 99% w/w lipid, about 20 to about 40% w/w phospholipid, and about 0.5 to about 5% w/w ganglioside, or
(j) about 80 to about 99% w/w lipid, about 60 to about 80% w/w phospholipid, and about 0.5 to about 5% w/w ganglioside, or
(k) about 15 to about 45% w/w lipid, about 8 to about 25% w/w phospholipid, and about 0.1 to about 5 % w/w ganglioside, or
(l) about 15 to about 45% w/w lipid, about 8 to about 25% w/w phospholipid, about 1 to about 5% w/w phosphatidylcholine, about 1 to about 5% w/w phosphatidylinositol, about 2 to about 8% w/w phosphatidylserine, about 2 to about 8% w/w phosphatidylethanolamine, about 0.5 to about 5% w/w sphingomyelin, and about 0.1 to about 5% w/w ganglioside, or
(m) about 50 to about 99% w/w lipid, about 15 to about 60% w/w phospholipid, and about 1 to about 10% w/w ganglioside, or
(n) about 50 to about 99% w/w lipid, about 15 to about 60% w/w phospholipid, about 1 to about 10% w/w phosphatidylcholine, about 1 to about 15% w/w phosphatidylinositol, about 1 to about 20% w/w phosphatidylserine, about 1 to about 20% w/w phosphatidylethanolamine, about 1 to about 10% w/w sphingomyelin, and about 0.1 to about 10% w/w ganglioside.
